# FASCICULE DE BREVET EUROPEEN

(11) **EP 1 511 771 B1**
(45) Date de publication et mention de la délivrance du brevet: **07.01.2009**
(21) Numéro de dépôt: 03760023.6
(22) Date de dépôt: 12.06.2003
(51) Int. Cl.: C07K 16/32, C07K 16/40, A61K 39/395, G01N 33/577, A61P 35/00

(54) **ANTICORPS MONOCLONAL ANTI-AURORA-A, SON PROCEDE D OBTENTION, ET SES UTILISATIONS DANS LE DIAGNOSTIC ET LE TRAITEMENT DES CANCERS**
MONOKLONALER ANTIKÖRPER GEGEN AURORA-A, SEINE HERSTELLUNGSVERFAHREN UND SEINE VERWENDUNG ZUR DIAGNOSE UND ZUR BEHANDLUNG VON KREBS
ANTI-AURORA-A MONOCLONAL ANTIBODY, METHOD FOR OBTAINING SAME, AND USES THEREOF FOR DIAGNOSING AND TREATING CANCERS

(30) Priorité: 12.06.2002 FR 0207212
(43) Date de publication de la demande: 09.03.2005
(73) Titulaire: CENTRE NATIONAL DE LA RECHERCHE SCIENTIFIQUE (CNRS), 75794 Paris Cedex 16 (FR); Etablissement Francais du Sang-Bretagne, 35016 Rennes Cédex (FR)
(72) Inventeur: PRIGENT, Claude, F-35235 Thorigne-Fouilard (FR); MARTIN, Anne, F-35700 Rennes (FR)
(74) Mandataire: Grosset-Fournier, Chantal Catherine
(86) Numéro de dépôt international: PCT/FR2003/001772
(87) Numéro de publication internationale: WO 2003/106500

(56) Documents cités:
- WO-A-97/22702
- US-A- 5 962 312
- TANAKA TAKUJI ET AL: "Centrosomal kinase AIK1 is overexpressed in invasive ductal carcinoma of the breast." CANCER RESEARCH, vol. 59, no. 9, 1 mai 1999 (1999-05-01), pages 2041-2044, XP002230542 ISSN: 0008-5472 cité dans la demande
- ARLOT-BONNEMAINS Y ET AL: "Identification of a functional destruction box in the Xenopus laevis aurora-A kinase pEg2" FEBS LETTERS, ELSEVIER SCIENCE PUBLISHERS, AMSTERDAM, NL, vol. 508, no. 1, 9 novembre 2001 (2001-11-09), pages 149-152, XP004322295 ISSN: 0014-5793
- CREMET JEAN YVES ET AL: "Preparation and characterization of a human aurora-A kinase monoclonal antibody." MOLECULAR AND CELLULAR BIOCHEMISTRY, vol. 243, no. 1-2, janvier 2003 (2003-01), pages 123-131, XP008023170 ISSN: 0300-8177 (ISSN print)

### LISTE DE SEQUENCES

<110> CNRS
<120> ANTICORPS MONOCLONAL ANTI-AURORA-A, SON PROCEDE D'OBTENTION, ET SES UTILISATIONS DANS LE DIAGNOSTIC ET LE TRAITEMENT DES CANCERS
<130> IFB 01 BN CNR AURA
<140>
   <141>
<160> 2
<170> PatentIn Ver. 2.1
<210> 1
   <211> 2253
   <212> ADN
   <213> Homo sapiens
<220>
   <221> CDS
   <222> (257)..(1495)
<400> 1
   ggaagacttg ggtccttggg tcgcaggtgg gagccgacgg gtgggtagac cgtgggggat 60
   atctcagtgg cggacgagga cggcggggac aaggggcggc tggtcggagt ggcggagcgt 120
   caagtcccct gtcggttcct ccgtccctga gtgtccttgg cgctgccttg tgcccgccca 180
   gcgcctttgc atccgctcct gggcaccgag gcgccctgta ggatactgct tgttacttat 240
   tacagctaga ggcatc atg gac cga tct aaa gaa aac tgc att tca gga cct 292
                     Met Asp Arg Ser Lys Glu Asn Cys Ile Ser Gly Pro
                      1               5                  10
   gtt aag gct aca gct cca gtt gga ggt cca aaa cgt gtt ctc gtg act   340
   Val Lys Ala Thr Ala Pro Val Gly Gly Pro Lys Arg Val Leu Val Thr
            15                  20                  25
   cag caa att cct tgt cag aat cca tta cct gta aat agt ggc cag gct   388
   Gln Gln Ile Pro Cys Gln Asn Pro Leu Pro Val Asn Ser Gly Gln Ala
        30                  35                  40
   cag cgg gtc ttg tgt cct tca aat tct tcc cag cgc gtt cct ttg caa   436
   Gln Arg Val Leu Cys Pro Ser Asn Ser Ser Gln Arg Val Pro Leu Gln
    45                  50                  55                  60
   gca caa aag ctt gtc tcc agt cac aag ccg gtt cag aat cag aag cag   484
   Ala Gln Lys Leu Val Ser Ser His Lys Pro Val Gln Asn Gln Lys Gln
                    65                  70                  75
   aag caa ttg cag gca acc agt gta cct cat cct gtc tcc agg cca ctg   532
   Lys Gln Leu Gln Ala Thr Ser Val Pro His Pro Val Ser Arg Pro Leu
                80                  85                  90
   aat aac acc caa aag agc aag cag ccc ctg cca tcg gca cct gaa aat    580
   Asn Asn Thr Gln Lys Ser Lys Gln Pro Leu Pro Ser Ala Pro Glu Asn
            95                 100                 105
   aat cct gag gag gaa ctg gca tca aaa cag aaa aat gaa gaa tca aaa    628
   Asn Pro Glu Glu Glu Leu Ala Ser Lys Gln Lys Asn Glu Glu Ser Lys
       110                 115                 120
   aag agg cag tgg gct ttg gaa gac ttt gaa att ggt cgc cct ctg ggt    676
   Lys Arg Gln Trp Ala Leu Glu Asp Phe Glu Ile Gly Arg Pro Leu Gly
   125                 130                 135                 140
   aaa gga aag ttt ggt aat gtt tat ttg gca aga gaa aag caa agc aag    724
   Lys Gly Lys Phe Gly Asn Val Tyr Leu Ala Arg Glu Lys Gln Ser Lys
                   145                 150                 155
   ttt att ctg gct ctt aaa gtg tta ttt aaa gct cag ctg gag aaa gcc    772
   Phe Ile Leu Ala Leu Lys Val Leu Phe Lys Ala Gln Leu Glu Lys Ala
               160                 165                 170
   gga gtg gag cat cag ctc aga aga gaa gta gaa ata cag tcc cac ctt    820
   Gly Val Glu His Gln Leu Arg Arg Glu Val Glu Ile Gln Ser His Leu
           175                 180                 185
   cgg cat cct aat att ctt aga ctg tat ggt tat ttc cat gat gct acc    868
   Arg His Pro Asn Ile Leu Arg Leu Tyr Gly Tyr Phe His Asp Ala Thr
       190                 195                 200
   aga gtc tac cta att ctg gaa tat gca cca ctt gga aca gtt tat aga    916
   Arg Val Tyr Leu Ile Leu Glu Tyr Ala Pro Leu Gly Thr Val Tyr Arg
   205                 210                 215                 220
   gaa ctt cag aaa ctt tca aag ttt gat gag cag aga act gct act tat    964
   Glu Leu Gln Lys Leu Ser Lys Phe Asp Glu Gln Arg Thr Ala Thr Tyr
                   225                 230                 235
   ata aca gaa ttg gca aat gcc ctg tct tac tgt cat tcg aag aga gtt    1012
   Ile Thr Glu Leu Ala Asn Ala Leu Ser Tyr Cys His Ser Lys Arg Val
               240                 245                 250
   att cat aga gac att aag cca gag aac tta ctt ctt gga tca gct gga    1060
   Ile His Arg Asp Ile Lys Pro Glu Asn Leu Leu Leu Gly Ser Ala Gly
           255                 260                 265
   gag ctt aaa att gca gat ttt ggg tgg tca gta cat gct cca tct tcc    1108
   Glu Leu Lys Ile Ala Asp Phe Gly Trp Ser Val His Ala Pro Ser Ser
       270                 275                 280
   agg agg acc act ctc tgt ggc acc ctg gac tac ctg ccc cct gaa atg    1156
   Arg Arg Thr Thr Leu Cys Gly Thr Leu Asp Tyr Leu Pro Pro Glu Met
   285                 290                 295                 300
   att gaa ggt cgg atg cat gat gag aag gtg gat ctc tgg agc ctt gga    1204
   Ile Glu Gly Arg Met His Asp Glu Lys Val Asp Leu Trp Ser Leu Gly
                   305                 310                 315
   gtt ctt tgc tat gaa ttt tta gtt ggg aag cct cct ttt gag gca aac    1252
   Val Leu Cys Tyr Glu Phe Leu Val Gly Lys Pro Pro Phe Glu Ala Asn
               320                 325                 330
   aca tac caa gag acc tac aaa aga ata tca cgg gtt gaa ttc aca ttc   1300
   Thr Tyr Gln Glu Thr Tyr Lys Arg Ile Ser Arg Val Glu Phe Thr Phe
           335                 340                 345
   cct gac ttt gta aca gag gga gcc agg gac ctc att tca aga ctg ttg   1348
   Pro Asp Phe Val Thr Glu Gly Ala Arg Asp Leu Ile Ser Arg Leu Leu
       350                 355                 360
   aag cat aat ccc agc cag agg cca atg ctc aga gaa gta ctt gaa cac   1396
   Lys His Asn Pro Ser Gln Arg Pro Met Leu Arg Glu Val Leu Glu His
   365                 370                 375                 380
   ccc tgg atc aca gca aat tca tca aaa cca tca aat tgc caa aac aaa   1444
   Pro Trp Ile Thr Ala Asn Ser Ser Lys Pro Ser Asn Cys Gln Asn Lys
                   385                 390                 395
   gaa tca gct agc aaa cag tct tag gaa tcg tgc agg ggg aga aat cct   1492
   Glu Ser Ala Ser Lys Gln Ser
               400
   tga gccagggctg ccatataacc tgacaggaac atgctactga agtttatttt        1545
   accattgact gctgccctca atctagaacg ctacacaaga aatatttgtt ttactcagca 1605
   ggtgtgcctt aacctcccta ttcagaaagc tccacatcaa taaacatgac actctgaagt 1665
   gaaagtagcc acgagaattg tgctacttat actggttcat aatctggagg caaggttcga 1725
   ctgcagccgc cccgtcagcc tgtgctaggc atggtgtctt cacaggaggc aaatccagag 1785
   cctggctgtg gggaaagtga ccactctgcc ctgaccccga tcagttaagg agctgtgcaa 1845
   taaccttcct agtacctgag tgagtgtgta acttattggg ttggcgaagc ctggtaaagc 1905
   tgttggaatg agtatgtgat tctttttaag tatgaaaata aagatatatg tacagacttg 1965
   tattttttct ctggtggcat tcctttagga atgctgtgtg tctgtccggc accccggtag 2025
   gcctgattgg gtttctagtc ctccttaacc acttatctcc catatgagag tgtgaaaaat 2085
   aggaacacgt gctctacctc catttaggga tttgcttggg atacagaaga ggccatgtgt 2145
   ctcagagctg ttaagggctt atttttttaa aacattggag tcatagcatg tgtgtaaact 2205
   ttaaatatgc aaataaataa gtatctatgt ctaaaaaaaa aaaaaaaa              2253
<210> 2
   <211> 403
   <212> PRT
   <213> Homo sapiens
<400> 2

## Description

La présente invention a pour objet un anticorps monoclonal dirigé contre la kinase aurora-A des mammifères, son procédé d'obtention, ainsi que ses utilisations dans le cadre du diagnostic ou du pronostic de cancers, et dans des compositions pharmaceutiques dans le cadre du traitement des cancers.

La protéine kinase aurora-A est un oncogène, sa surexpression dans des cellules Rat-1 suffit à provoquer l'apparition d'un phénotype transformé et l'implantation de ces cellules transformées dans des souris immunodéficientes induit l'apparition de tumeurs. (Bischoff et al., 1998 ; Zhou et al., 1998). Le gène codant pour cette kinase est localisé sur le chromosome 20 en 20q13, amplicon fréquemment détecté dans de nombreuses tumeurs (sein, colon, cancer gastriques).

La surexpression de la protéine kinase aurora-A a été observée dans de nombreuses tumeurs. De manière très intéressante la présence de cette kinase en quantité anormale n'est pas corrélée à une prolifération détectée par coloration avec un marqueur spécifique de la prolifération tel que PCNA. Aurora-A est donc un marqueur spécifique de l'aspect tumoral des cellules (Tanaka et al., 1999 ; Takahashi et al., 2000).

Aurora-A fait partie d'une famille multigénique de protéines kinases appelées aurora, elle comporte trois membres : aurora-A (décrite précédemment) aurora-B (Prigent et al., 1999) et aurora-C (Bernard et al., 1998). Bien que seule aurora-A ait un réel pouvoir oncogène les deux autres kinases ont également été retrouvées surexprimées dans les mêmes tumeurs (Giet et Prigent, 1999).

L'amplification du gène codant pour aurora-A est associée à la présence d'une activité anormalement élevée de la protéine kinase dans ces tumeurs. De plus la surexpression ectopique de cette kinase dans des cellules en culture suffit à provoquer l'apparition d'un phénotype transformé, ces cellules transplantées dans des souris immunodéficientes induisent l'apparition de tumeurs.

La surexpression de la kinase aurora-A est très étroitement liée à l'état cancéreux d'une cellule. Cette surexpression de la kinase aurora-A induit une polyploïdie des cellules et provoque une amplification des centrosomes, deux événements préfigurant un mauvais pronostic du cancer du sein par exemple.

Il est donc important de pouvoir mesurer précisément l'expression de cette kinase dans les pathologies cancéreuses, tant au niveau ARNm que protéine.

Or la mesure de l'expression de la protéine kinase aurora-A dépend entièrement de l'utilisation d'un bon anticorps monoclonal.

Toutefois, aucun anticorps monoclonal suffisamment spécifique dirigé contre la protéine kinase humaine aurora-A n'a pu être obtenu jusqu'à présent, et n'est disponible dans le commerce.

La présente invention a pour but de fournir un anticorps monoclonal anti-aurora-A fiable, se liant à cette protéine avec une spécificité et une sensibilité suffisante pour envisager son utilisation à des fins de recherche expérimentale, ainsi que dans le domaine du diagnostic, du pronostic et du traitement des cancers.

L'invention concerne un anticorps monoclonal anti-aurora-A reconnaissant spécifiquement la kinase aurora-A humaine et murine, et ayant les propriétés suivantes :
* il peut se fixer sur les membranes contenant la protéine aurora-A humaine ou murine,
* il permet de détecter, et, le cas échéant, de purifier, la protéine aurora-A humaine et murine par immunoprécipitation,
* il permet la coloration des tissus biologiques où est sécrétée la protéine aurora-A et,
* il n'inhibe pas l'activité enzymatique de la protéine aurora-A humaine et murine, ledit anticorps monoclonal étant tel qu'obtenu par :
   - cinq injections espacées de quinze jours à des souris de protéine kinase aurora-A recombinante produite par des bactéries *E. coli* transformées avec un vecteur d'expression bactérien dans le génome duquel a été inséré l'ADNc humain codant pour aurora-A, sacrifice desdites souris, et fusion entre des cellules de la rate de ces souris et des cellules de hamster immortalisées en culture pour obtenir des hybridomes,
   - criblage des hybridomes produisant un anticorps capable d'immunoprécipiter la protéine recombinante utilisée pour l'immunisation des souris lors de l'étape précédente, et récupération des hybridomes positifs après ce premier crible,
   - criblage des hybridomes récupérés à l'étape précédente, produisant un anticorps capable d'immunoprécipiter la protéine endogène aurora-A à partir d'un extrait de cellules humaines HeLa en culture, et récupération des hybridomes positifs après ce deuxième crible,
   - criblage des hybridomes récupérés à l'étape précédente, produisant un anticorps capable de reconnaître en immunofluorescence indirecte les centrosomes et les pôles du fuseau mitotique de cellules humaines en culture, et récupération des hybridomes positifs après ce troisième crible,
   - criblage des hybridomes récupérés à l'étape précédente, produisant un anticorps capable d'immunoprécipiter la protéine endogène aurora-A de souris à partir d'un extrait de cellules murines en culture, et récupération des hybridomes positifs après ce quatrième crible,
   - criblage des hybridomes récupérés à l'étape précédente, produisant un anticorps capable de reconnaître en immunofluorescence indirecte les centrosomes et les pôles du fuseau mitotique de cellules murines en culture,
   - récupération et purification par clonage d'un hybridome positif après l'étape de criblage précédente, et produisant un anticorps monoclonal possédant l'ensemble des propriétés définies ci-dessus.

A ce titre, l'invention a plus particulièrement pour objet un anticorps monoclonal tel que défini ci-dessus, encore désigné anticorps 35C1, ledit anticorps étant secrété par l'hybridome déposé le 12 juin 2003 à la Collection Nationale de Cultures de Microorganismes (CNCM) de l'Institut Pasteur sous le numéro I-3050.

L'invention a également pour objet l'utilisation d'un anticorps monoclonal tel que défini ci-dessus, et plus particulièrement de l'anticorps 35C1 susmentionné, pour la mise en oeuvre d'une méthode de diagnostic *in vitro,* ou de pronostic de cancers chez l'homme ou l'animal.

L'invention a plus particulièrement pour objet l'utilisation d'un anticorps monoclonal tel que défini ci-dessus, et plus particulièrement de l'anticorps 35C1 susmentionné, pour la mise en oeuvre d'une méthode de diagnostic *in vitro,* ou de pronostic, de tumeurs solides, tels que les cancers du sein, les cancers gastriques et les cancers colorectaux.

L'invention concerne également l'utilisation susmentionnée d'un anticorps monoclonal tel que défini ci-dessus, et plus particulièrement de l'anticorps 35C1 susmentionné, en association avec un marqueur de prolifération cellulaire, tel qu'un marqueur de la protéine PCNA (Tanaka et al., 1999 ; Takahashi et al., 2000).

L'invention a également pour objet toute méthode de diagnostic *in vitro,* ou de pronostic, de cancers tels que définis ci-dessus, chez l'homme ou l'animal, caractérisée en ce qu'elle comprend :
- la mise en présence d'un anticorps monoclonal tel que défini ci-dessus, et plus particulièrement de l'anticorps 35C1 susmentionné, avec un échantillon biologique prélevé chez un individu, ledit anticorps étant le cas échéant fixé sur un support solide,
- la détection, et le cas échéant la quantification, de la protéine aurora-A susceptible d'être présente dans l'échantillon biologique à l'aide de réactifs marqués, notamment d'anticorps marqués, reconnaissant soit l'anticorps monoclonal lié à ladite protéine aurora-A, soit la protéine aurora-A liée audit anticorps monoclonal dans les complexes formés lors de l'étape précédente entre l'anticorps monoclonal et la protéine aurora-A susceptible d'être présente dans l'échantillon biologique, et ce, le cas échéant, après rinçage approprié du support solide.

Avantageusement, dans le cadre de la méthode susmentionnée, la détermination d'une quantité de protéine aurora-A inférieure ou supérieure à un seuil physiologique déterminé en fonction de l'échantillon biologique, témoigne respectivement d'un bon ou d'un mauvais pronostic du cancer diagnostiqué.

L'invention a également pour objet, un kit pour la mise en oeuvre d'une méthode de diagnostic définie ci-dessus, caractérisé en ce qu'il comprend :
- un anticorps monoclonal tel que défini ci-dessus, et plus particulièrement l'anticorps 35C1 susmentionné,
- le cas échéant, un marqueur de prolifération cellulaire, tel qu'un marqueur de la protéine PCNA, notamment un anticorps anti-PCNA.

L'invention concerne également l'utilisation d'un anticorps monoclonal tel que défini ci-dessus, et plus particulièrement de l'anticorps 35C1 susmentionné, pour la préparation de médicaments destinés au traitement de cancers, tels que les cancers du sein, les cancers colorectaux et les cancers gastriques.

A ce titre, l'invention a plus particulièrement pour objet toute composition pharmaceutique, contenant un anticorps monoclonal tel que défini ci-dessus, et plus particulièrement l'anticorps 35C1 susmentionné, en association avec un véhicule pharmaceutiquement acceptable.

L'invention a également pour objet l'utilisation d'un anticorps monoclonal tel que défini ci-dessus, et plus particulièrement de l'anticorps 35C1 susmentionné, pour la mise en oeuvre d'une méthode de criblage d'inhibiteurs de la kinase aurora-A dans laquelle la baisse de l'activité de cette kinase est mesurée à l'aide dudit anticorps.

L'invention a plus particulièrement pour objet toute méthode de criblage d'inhibiteurs de la kinase aurora A caractérisée en ce qu'elle comprend les étapes suivantes :
- le traitement de cellules, telles que des lignées dérivées de différents cancers (sein, colon etc...), par l'inhibiteur testé,
- immunoprécipitation de la protéine kinase aurora-A à l'aide d'un anticorps monoclonal tel que défini ci-dessus, et plus particulièrement de l'anticorps 35C1 susmentionné, et mesure de l'activité kinase, notamment selon la méthode décrite dans le paragraphe 3. g) ci-après.

L'invention concerne également le procédé de préparation d'un anticorps monoclonal tel que défini ci-dessus, et plus particulièrement de l'anticorps 35C1 susmentionné, caractérisé en ce qu'il comprend les étapes suivantes:
- cinq injections espacées de quinze jours à des souris de protéine kinase aurora-A recombinante produite par des bactéries *E. coli* transformées avec un vecteur d'expression bactérien dans le génome duquel a été inséré l'ADNc humain codant pour aurora-A, sacrifice desdites souris, et fusion entre des cellules de la rate de ces souris et des cellules de hamster immortalisées en culture pour obtenir des hybridomes,
- criblage des hybridomes produisant un anticorps capable d'immunoprécipiter la protéine recombinante utilisée pour l'immunisation des souris lors de l'étape précédente, et récupération des hybridomes positifs après ce premier crible,
- criblage des hybridomes récupérés à l'étape précédente, produisant un anticorps capable d'immunoprécipiter la protéine endogène aurora-A à partir d'un extrait de cellules humaines HeLa en culture, et récupération, des hybridomes positifs après ce deuxième crible,
- criblage des hybridomes récupérés à l'étape précédente, produisant un anticorps capable de reconnaître en immunofluorescence indirecte les centrosomes et les pôles du fuseau mitotique de cellules humaines en culture, et récupération des hybridomes positifs après ce troisième crible,
- criblage des hybridomes récupérés à l'étape précédente, produisant un anticorps capable d'immunoprécipiter la protéine endogène aurora-A de souris à partir d'un extrait de cellules murines en culture, et récupération des hybridomes positifs après ce quatrième crible,
- criblage des hybridomes récupérés à l'étape précédente, produisant un anticorps capable de reconnaître en immunofluorescence indirecte les centrosomes et les pôles du fuseau mitotique de cellules murines en culture,
- récupération et purification par clonage d'un hybridome positif après l'étape de criblage précédente, et produisant un anticorps monoclonal possédant l'ensemble des propriétés définies ci-dessus.

L'invention sera davantage illustrée à l'aide de la description détaillée de l'anticorps monoclonal 35C1 défini ci-dessus et de son procédé d'obtention.

Le cDNA humain codant pour aurora-A (SEQ ID NO : 1) a été inséré dans un vecteur d'expression bactérien (pET29 Novagene).

La protéine kinase a été produite dans des bactéries BL21 (DE3)pLysS et purifiée par chromatographie d'affinité sur colonne Ni-NTA-agarose (Qiagen).

La protéine purifiée au laboratoire a ensuite été injectée à des souris (BALB/c).

Après cinq injections espacées de 15 jours la souris a été sacrifiée et une fusion a été effectuée entre des cellules de la rate de la souris et des cellules de hamster immortalisées en culture pour obtenir des hybridomes.

Les hybridomes obtenus au nombre de 960 ont alors été testés pour leur capacité à produire un anticorps reconnaissant en western blot la protéine utilisée pour l'immunisation.

Les hybridomes positifs après ce premier crible ont alors été testés en western blot pour leur capacité à reconnaître la protéine endogène aurora-A à partir d'un extrait de cellules humaines HeLa en culture.

Les hybridomes positifs après ce deuxième crible ont été testés pour leur capacité à reconnaître en immunofluorescence indirecte les centrosomes et les pôles du fuseau mitotiques de cellules humaines en culture.

Les hybridomes positifs après ce troisième crible ont alors été testés en western blot pour leur capacité à reconnaître la protéine endogène aurora-A de souris à partir d'un extrait de cellules murines en culture.

Les hybridomes positifs après ce quatrième crible ont été testés pour leur capacité à reconnaître en immunofluorescence indirecte les centrosomes et les pôles du fuseau mitotiques de cellules murines en culture.

Un hybridome répondant à tous ces critères a été retenu et cloné pour obtenir un clone pur. Ce clone a été baptisé 35C1.

Il sécrète un anticorps monoclonal anti-aurora-A qui reconnaît la kinase aurora-A humaine et murine.

Cet anticorps monoclonal anti-aurora-A reconnaissant spécifiquement la kinase aurora-A humaine et murine a les propriétés suivantes :
- il peut-être utilisé en Western-blot (immunodétection indirecte de la protéines sur membrane de nitrocellulose ou nylon)
- il permet de localiser la protéine dans des cellules en culture par immunodétection indirecte
- il.n'inhibe pas l'activité enzymatique de la kinase in vitro
- il permet de purifier la kinase aurora-A d'extrait acellulaire par immunoprécipitation
- puisqu'il n'inhibe pas l'activité kinase de aurora-A il peut être utilisé pour doser l'activité kinase dans des extraits protéiques préparés à partir de tissus présentant des pathologies.

### 1) Purification de la protéine aurora-A recombinante

Le cDNA codant pour la kinase aurora-A humaine a été cloné dans le vecteur d'expression bactérien pET29 (fournisseur Novagen) qui permet de produire une protéine recombinante contenant 6 résidus histidine supplémentaires. La souche de bactérie *E. coli* BL21(DE3) pLysS (fournisseur Promega) qui est déficiente en protéase et qui s'autolyse par production de lysozyme après décongélation (toutes ces propriétés facilitent la purification de protéines) a été utilisée. La surexpression de la protéine aurora-A-(His)6 dans les bactéries en phase de croissance (DO₆₀₀ = 0,6) est induite à 22°C par addition de 1 mM IPTG (Isopropyl-β-D-thiogalactoside) pendant 4 heures. Les bactéries sont ensuite lysées à 4°C en utilisant en plus de leur propriété autolytique, du lysozyme et des ultrasons. La protéine aurora-A-(His)6 est ensuite purifiée par chromatographie d'affinité sur colonne de nickel (Ni-NTA-agarose (fournisseur Qiagen). La protéine est éluée par 250 mM imidazole suivant les instructions Qiagen. La protéine purifiée est ensuite passée sur centricon YM-10 (fournisseur Millipore) pour la placer dans une solution de PBS (NaCl 136 mM, KCl 26 mM, Na₂HPO₄ 2 mM, KH₂PO₄ 2 mM, pH 7,2). Des fractions de 15 µg de protéine ont été préparées, lyophilisées et conservées à 4°C.

### 2) Immunisation de la souris

Une souris BALB/c a été immunisée par voie intra-péritonéale avec 15 µg de protéine aurora-A recombinante diluée dans 50% d'adjuvant de Freund complet (fournisseur Sigma). La souris a ensuite été injectée deux fois 15 µg de protéine aurora-A recombinant diluée dans 50% d'adjuvant de Freund incomplet avec trois semaines d'intervalle. Lorsque des anticorps anti-aurora-A ont été détectés dans le sang de la souris, elle a été sacrifiée et la rate prélevée. Des cellules en suspension ont été obtenues à partir de cette rate par homogénéisation avec un Dounce.

Ces cellules de rate ont été fusionnées avec des cellules SP2/O-Ag14 provenant d'un myélome murin et obtenues auprès de l'ECACC (Shulman et al., 1978). Une fusion a été effectuée entre 100.10⁶ cellules de rate et 20.10⁶ cellules SP2/O-Ag14 dans 50% de polyéthylène glycol 1500 (fournisseur Roche) pendant 90 mn à 37°C. Les cellules ont ensuite été distribuées dans des boites 10 x 96 puits contenant un milieu de sélection HAT (fournisseur Sigma Chemicals).

### 3) Crible des hybridomes

### a) ELISA

100 µl de PBS contenant 4 µg/ml de protéine recombinante aurora-A ont été déposés dans chaque puits de plaques Elisa (plaques 96 puits) et incubé 36 heures a 4°C. Après deux lavages avec du PBS les puits sont remplis de PBS contenant 3% de BSA (Albumine Sérique Bovine , fournisseur Sigma) et les plaques sont incubées une nuit à 4°C. Le lendemain 100 µl de chaque surnageant de fusion est transféré dans ces plaques 96 puits contenant aurora-A recombinante. Les plaques sont incubées à température ambiante pendant 2 heures. Après deux lavages avec du PBS/BSA, les plaques sont incubées avec un anticorps anti-souris couplé à la phosphatase (Sigma Biochemical). Les puits sont ensuite lavés deux fois avec du PBS et une fois avec une solution AP (100 mM Tris pH 9.5, 100 mM NaCl and 5 mM MgCl₂). La présence d'un anticorps monoclonal est détectée après remplissage des puits avec 50 µl de solution AP contenant le substrat synthétique de phosphatase (Nitro-4-phenyl phosphate disodique hexahydrate salt) à 5 mg/ml (fournisseur Merck) et par l'apparition d'une coloration jaune dans le puits.

### b) Western blot contre la protéine recombinante

Dix plaques 96 puits (8x12) ont été analysées par tests ELISA sans donner de résultats très reproductifs. Ces plaques ont alors été testées par Western blot effectués de la manière suivante. La protéine recombinante aurora-A a été soumise à une électrophorèse sur gel de polyacrylamide-SDS et transférée sur membrane de nitrocellulose selon la technique décrite précédemment (Roghi et al., 1998). Les membranes ont été découpées pour isoler la région correspondant à l'emplacement ou migrait la protéine aurora-A. Les bouts de membranes ont été bloquées par incubation dans une solution de TBST (20 mM Tris-HCl pH 7.5, 150 mM NaCl, 0.05% Tween 20) contenant 5% de lait pendant 2 heures à 4°C. Chaque bout de membrane a ensuite été incubé avec les surnageants de cellule dilués au 1:100 dans une solution de TBST contenant 2.5% de lait pendant 1 h à 4°C. Les immuno-complexes ont été identifiés en utilisant soit un second anticorps anti-souris couplé à la peroxidase ou à la phosphatase (fournisseur Sigma Chemicals) à la dilution préconisée par le fabriquant. La révélation de la réaction a été effectuée par la technique de chemiluminescence pour la péroxidase (fournisseur Amersham Pharmacia Biotech) selon les indications du fournisseur ou par colorimétrie pour la phosphatase en utilisant les deux substrats NBT/BCIP (fournisseur Sigma Chemicals) selon les indications du fournisseur.

Les puits de chaque plaque ont été regroupés par pools de 8 correspondant à chaque colonne de chaque plaque. La présence de monoclonaux a été analysée dans chaque pool par Western blot contre la protéine recombinante aurora-A. Sur les 120 pools testés seuls 19 ont donné une réponse positive.

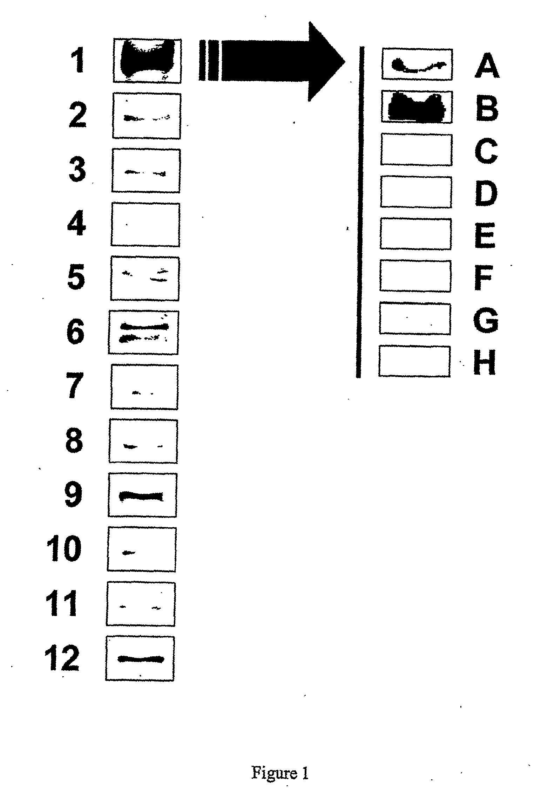

Chacun des 8 puits correspondant à chaque pool positif a été testé séparément par la même technique de Western blot dans le but d'identifier quel(s) puits contenait(ent) des anticorps. La figure 1 montre un exemple de résultats obtenus avec le pool numéro 2, dans ce cas particulier seuls les puits A et B contenaient des anticorps, le puits ayant été retenu.

Sur les 120 pools testés seuls 19 ont été retenus parce qu'ils donnaient une très forte réponse positive. Dans ces 19 pools seuls 23 puits contenaient des anticorps dirigés contre la protéine aurora-A recombinante.

### c) Western blot contre la protéine aurora-A humaine endogène

La même technique de Western blot a cette fois été utilisée pour identifier les surnageants capable de reconnaître la protéine aurora-A parmi toutes les protéines d'un extrait acellulaire total préparé à partir de cellules humaines en culture.

Les cellules choisies sont des cellules HeLa. Les extraits ont été préparés à partir de boite de culture contenant environ 10⁶ cellules, les cellules ont été lysées dans leur boite par 1 ml de solution dite Laemmli correspondant à la solution de dépôt sur gel polyacrylamide-SDS (Laemmli 1970), la solution a été incubée 10 min à 90°C, soniquée et centrifugée, 10 µl du surnageant est déposé sur le gel.

Parmi les 23 surnageant ayant été sélectionnés précédemment seul 12 contenait un anticorps capable de reconnaître une protéine de 46 kD (taille attendue pour aurora-A) par Western blots effectués sur des extrait de cellules Hela.

### d) Immunofluorescence sur cellules humaines

Une étape supplémentaire a été introduite dans le crible pour sélectionner les anticorps qui étaient capable de décorer le centrosome dans des cellules humaines en culture. Le choix des cellules s'est porté la lignée cellulaire MCF7 dérivant d'un cancer du sein car la protéine aurora-A a été rapportée surexprimée dans ces cellules.

La technique utilisée est l'immunofluorescence indirecte. Les cellules sont cultivées sur des lamelles rondes en verre dans les boites 12 puits (fournisseur Corning Inc) pendant 48 heures. Les lamelles sont ensuite lavées par une solution de PBS et les cellules fixées par du méthanol froid (-20°C). Les cellules sont ensuite incubées 30 mn à température ambiante dans du PBS contenant de la BSA 3%. Après trois lavages par du PBS les lamelles sont incubées avec les surnageant d'hybridome dilués au 1:50 dans du PBS pendant 1 heure à température ambiante. Les cellules sont a nouveau lavées trois fois par du PBS et incubées à température ambiante pendant 1 heure avec un second anticorps anti-souris couplé à la fluorescéine « FITC » (fournisseur Sigma Chemicals). Les lamelles sont lavées trois fois par du PBS et les cellules montées entre lame et lamelle dans du Mowiol contenant de l'antifading. Les observations ont été effectuées avec un microscope fluorescent Leica DMRXA et les images prises avec une caméra noir et banc (COHU) ont été traitées par le logiciel Leica Qfish.

Sur les 12 surnageants retenus précédemment seuls 4 contenaient des anticorps capables de décorer les centrosomes et les pôles du fuseau mitotique des cellules MCF7. Cette localisation correspond exactement à celle attendue pour la kinase aurora-A.

### e) Western blot contre la protéine aurora-A murine endogène

Dans le but d'augmenter la sélectivité du crible nous avons testé les 4 surnageants contre la protéine orthologue de aurora-A chez la souris. Un premier crible a été effectué par Western blot contre des extraits acellulaires de cellules de souris en culture, cellules m-ICc12. Les extraits acellulaires ont été préparés comme pour les cellules humaines et le Western blots effectués de la même manière que précédemment. Deux des surnageant étaient capable de reconnaître une protéine de 46 kD (taille également attendue pour la kinase aurora-A murine)

### f) Immunofluorescence sur cellules murines

Nous avons contrôlé si les deux surnageants précédemment identifiés en Western blot étaient capable de décorer les centrosomes de cellules de souris en culture. Nous avons choisi les cellules LLC1 car elles présentent un index mitotique très élevé. Seul un des deux anticorps a été capable de localiser une protéine dans les centrosomes et aux pôles du fuseau mitotique, localisations attendues pour la protéine kinase aurora-A de souris.

### g) Dosage de l'activité kinase aurora-A

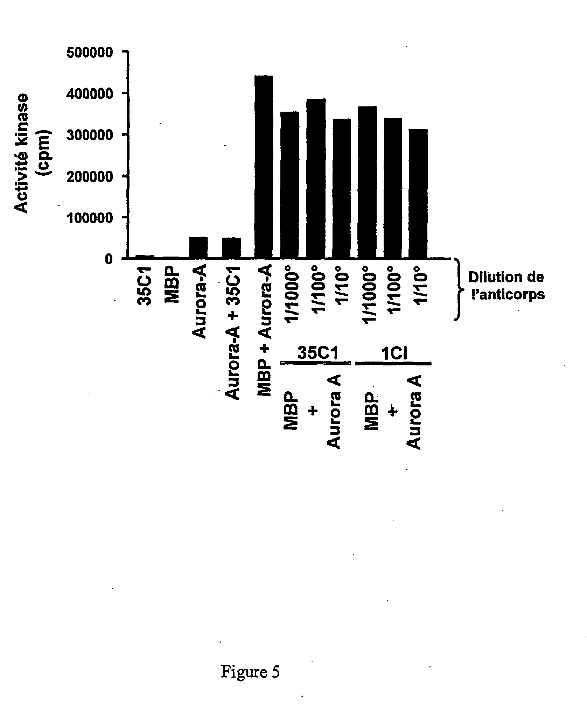

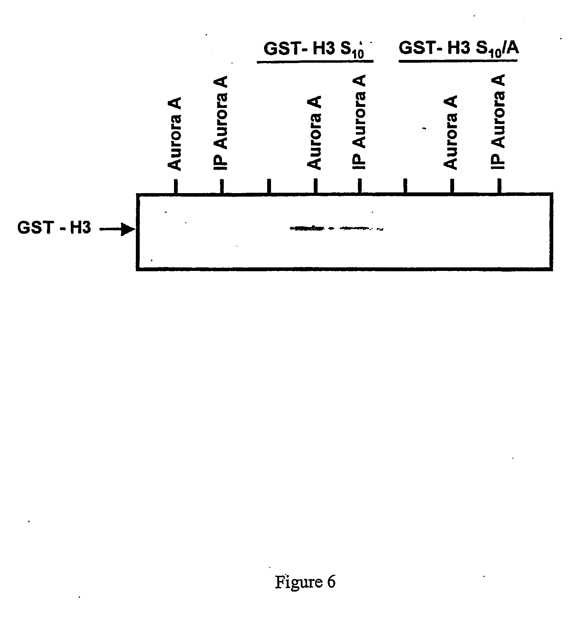

Les mesures de l'activité kinase aurora-A ont été effectuées dans 20 µl dé Tris-HCl 50 mM pH 7,5, NaCl 50 mM, DTT 1 mM, MgCl₂10 mM, et ATP 10 µM dont 1 µCi de [γ-³²P] ATP 3000 Ci/mmole (fournisseur Amersham Pharmacia Biotech) contenant 4 µg de protéine basique de la myéline (MBP) pour la figure 5 (fournisseur Sigma Chemicals) ou 10 µl d'un extrait de bactéries ayant produite la protéine GST-H3 pour la figure 6. Les réactions sont incubées à 37°C pendant 10 min. 10 µl de la réaction sont analysés soit en comptage (figure 5) soit après migration sur gel de polyacrylamide-SDS, séché et examiné en autoradiographie (figure 6).

### h) Clonage du monoclonal sélectionné

Le surnageant que nous avons sélectionné contenait un mélange hétérogène de cellules obtenus après la fusion. Nous avons repiqué ces cellules en effectuant une dilution limite et obtenu 20 clones. Le surnageant de ces 20 clones a été testé en Western blot contre la protéine recombinante aurora-A, 8 ont donné une réponse positive. Ces 8 surnageants ont été testés sur des extraits de cellules humaines HeLa, de cellules murines m-ICc12. Seuls deux surnageants ont été retenus.

Ces deux surnageants ont à nouveau été re-clonés par dilution limite et re-testés comme précédemment. Ce dernier clonage avait pour but de sélectionner un clone qui maintenait un niveau de production d'anticorps reproductible après repiquage.

Seul un des deux clone s'est avéré stable, il a été baptisé 35C1 et retenu pour stockage et production de monoclonal.

### i) Propriétés du monoclonal 35C1 (voir les figures)

L'anticorps reconnaît spécifiquement que la protéine kinase aurora-A humaine et murine en Western blot dans des extraits acellulaire totaux (figure 1).

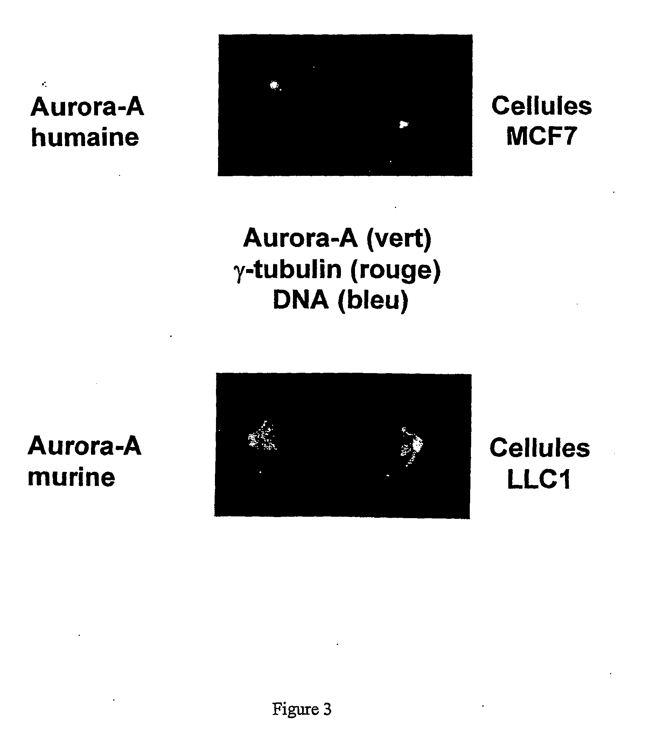

Il localise la protéine kinase aurora-A dans des cellules humaines et dans des cellules murines en culture (figure 3).

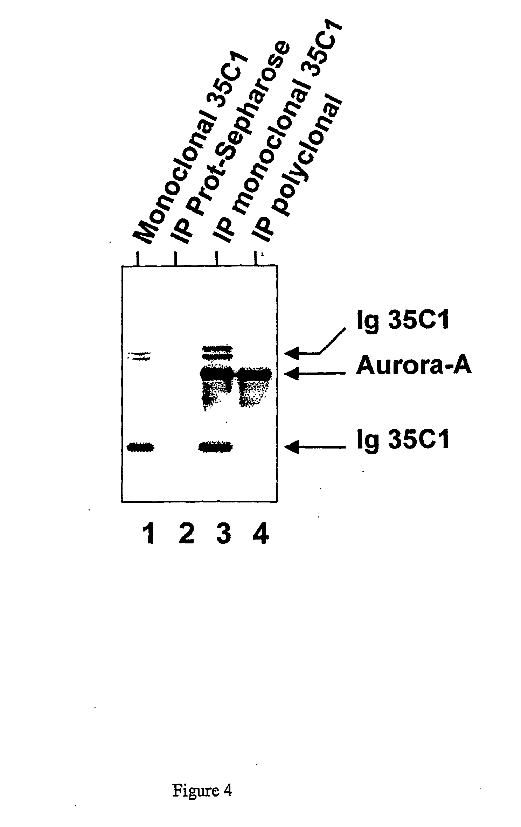

Il immunoprécipite la protéine aurora-A d'extraits de cellules humaine MCF7 (figure 4).

Il n'inhibe pas l'activité kinase de aurora-A (figure 5).

Il permet donc d'immuno-précipiter la protéine aurora-A et de mesurer son activité kinase alors qu'elle est toujours associée à l'anticorps (figure 6)

Ces propriétés du monoclonal 35C1 en font un outil de choix pour l'étude de la protéine kinase aurora-A.

Il peut être utilisé dans des méthodes de diagnostic et de pronostic de tumeurs solides. Le niveau d'expression de l'ARNm codant pour la protéine aurora-A est étroitement corrélée avec l'instabilité génétique des cellules de cancer du sein et avec un grade élevé de la tumeur (Miyoshi et al. 2001). Ceci a été très clairement établi pour le cancer du sein. Par contre en raison de l'absence d'anticorps monoclonal suffisamment spécifique, cette corrélation entre la quantité d'ARNm aurora-A et le grade du cancer n'a pas encore pu être vérifiée au niveau protéique. Le monoclonal 35C1 anti-aurora-A permettra ce type de mesures. Il permet d'une part de mesurer la quantité de protéine aurora-A (Western blot et immunohistochimie) et d'autre part de mesurer l'activité aurora-A (immunoprécipitation) dans des tumeurs, et ainsi de déterminer le seuil de la quantité d'aurora-A au-dessous et au-dessus duquel le pronostic d'un cancer déterminé sera bon ou mauvais respectivement.

Par ailleurs, l'anticorps 35C1 permet de tester l'efficacité d'inhibiteurs de l'activité kinase aurora-A in vivo. La protéine kinase aurora-A est immunoprécipitée de cellules HeLa par exemple préalablement traitées par l'inhibiteur et son activité mesurée *in vivo.* Ceci permet entre autre d'évaluer la stabilité des inhibiteurs in vivo.

### Références bibliographiques

Bernard M, Sanseau P, Henry C, Couturier A, Prigent C. Cloning of STK13, a third human protein kinase related to Drosophila aurora and budding yeast Ip11 that maps on chromosome 19q13.3-ter. Genomics. 1998 Nov 1;53(3):406-9.

Bischoff JR, Anderson L, Zhu Y, Mossie K, Ng L, Souza B, Schryver B, Flanagan P, Clairvoyant F, Ginther C, Chan CS, Novotny M, Slamon DJ, Plowman GD. A homologue of Drosophila aurora kinase is oncogenic and amplified in human colorectal cancers. EMBO J 1998 Jun 1;17(11):3052-65.

Giet R, Prigent C. Aurora/Ip11p-related kinases, a new oncogenic family of mitotic serine-threonine kinases. J Cell Sci 1999 Nov;112 ( Pt 21):3591-601.

Laemmli U.K. Cleavage of structural proteins during the assembly of the head of bacteriophage T4. Nature. 1970 Aug 15;227(259):680-5.

Miyoshi Y, Iwao K, Egawa C, Noguchi S. Association of centrosomal kinase STK15/BTAK mRNA expression with chromosomal instability in human breast cancers. Int J Cancer 2001 May 1;92(3):370-3.

Prigent C, Gill R, Trower M, Sanseau P. In silico cloning of a new protein kinase, Aik2, related to Drosophila Aurora using the new tool: EST Blast. In Silico Biol 1999;1(2): 123-8.

Roghi C, Giet R, Uzbekov R., Morin N, Chartrain I, Le Guellec R, Couturier A, Doree M, Philippe M, Prigent C. The Xenopus protein kinase pEg2 associates with the centrosome in a cell cycle-dependent manner, binds to the spindle microtubules and is involved in bipolar mitotic spindle assembly. J Cell Sci. 1998 Mar;111 (Pt 5):557-72.

Shulman M, Wilde CD, Kohler G. A better cell line for making hybridomas secreting specific antibodies. Nature 1978 Nov 16;276(5685):269-70.

Takahashi T, Futamura M, Yoshimi N, Sano J, Katada M, Takagi Y, Kimura M, Yoshioka T, Okano Y, Saji S. (2000) Centrosomal kinases, HsAIRK1 and HsAIRK3, are overexpressed in primary colorectal cancers. Jpn J Cancer Res 91(10):1007-14

Takahashi T, Futamura M, Yoshimi N, Sano J, Katada M, Takagi Y, Kimura M, Yoshioka T, Okano Y, Saji S. Centrosomal kinases, HsAIRK1 and HsAIRK3, are overexpressed in primary colorectal cancers. Jpn J Cancer Res 2000 Oct;91(10):1007-14.

Tanaka T, Kimura M, Matsunaga K, Fukada D, Mori H, Okano Y. Centrosomal kinase AIK1 is overexpressed in invasive ductal carcinoma of the breast. Cancer Res 1999 May 1;59(9):2041-4.

Zhou H, Kuang J, Zhong L, Kuo WL, Gray JW, Sahin A, Brinkley BR, Sen S. Tumour amplified kinase STK15/BTAK induces centrosome amplification, aneuploidy and transformation. Nat Genet. 1998 Oct;20(2):104-6.

### Légende des figures

Légende de la figure 1 : Crible des hybridomes par western blot. La protéine recombinante aurora-A purifiée a été déposée sur gel de polyacrylamide-SDS et transférée sur une membrane de nitrocellulose. La membrane a été colorée au rouge ponceau et la bande correspondant à aurora-A découopée. Chaque panneau correspond à un morceau de membrane avec aurora-A. Après fusion les cellules ont été distribuées dans des boites 96 puits. Pour cribler la présence de monoclonaux anti-aurora-A des aliquots des surnageants des puits de chaque colonne sont regroupés en pools, ceci pour chaque boite. Chaque pool est ensuite testé en western blot colonne de droite de 1 à 12. Lorsqu'un pool est considéré comme positif, ici le pool numéro 1, les surnageants de chaque puits constituant ce pool (de A à H) sont re-testé individuellement. Dans ce cas précis les puits A et B contenaient des anticorps, mais seul le puits B a été retenu.

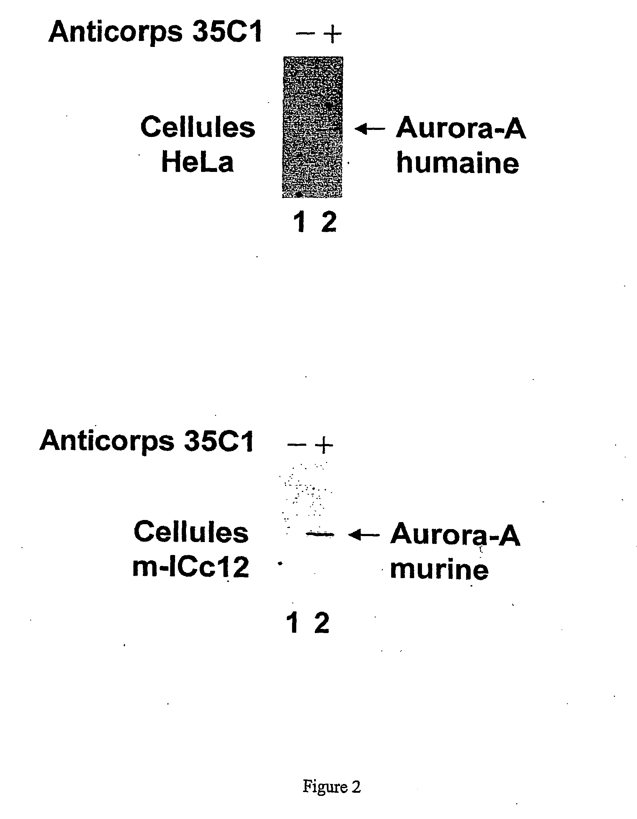

Légende figure 2 : Western blot. Les extraits acellulaires totaux sont séparés sur gel de polyacrylamide SDS et le gel est transféré sur membrane de nitrocellulose. Le puits 1 ne contient pas d'extrait et le puit 2 contient 10 µl d'extrait (correspondant à 10⁶ cellules par ml). L'anticorps est utilisé à une dilution de 1/100. Seule la protéine aurora-A de 46 kD est détectée.

Légende figure 3 : Immunodétection indirecte de aurora-A dans des cellules humaines et murines. Les cellules humaines sont des MCF7 et les cellules murines des LLC1. Sont détectés en immunofluorescence l'ADN par coloration DAPI (bleu), la γ-tubuline (rouge) et aurora-A (vert)

Légende figure 4: Immunoprécipitation de aurora-A. La protéine est immunoprécipitée par l'anticorps 35C1 couplé à la protéine A Sepharose. Les immunoprécipités sont séparés sur un gel de polyacrylamide-SDS, le gel est transféré et les immunocomplexes révélés avec le monoclonal 35C1. Puits 1 : l'anticorps 35C1 seul ; puits 2 : immunoprécipitation effectuée avec la protéine A Sepharose seule ; puits 3 : immunoprécipitation effectuée avec l'anticorps monoclonal 35C1 ; puits 4 : immunoprécipitation effectuée avec un anticorps préparé au laboratoire.

Légende figure 5 : Activité de la kinase recombinante aurora-A humaine purifiée mesurée en présence de l'anticorps monoclonal 35C1. L'anticorps 1C1 dirigé contre la protéine aurora-A de xénope et qui ne croise pas avec la protéine humaine est utilisé comme contrôle. L'activité kinase est mesurée en utilisant la MBP (Myelin Basic Protein) comme substrat.

Légende figure 6 : Activité de la protéine aurora-A endogène immunoprécipitée par l'anticorps 35C1 fixé sur des bille de protéine A Dynabeads. L'activité de la kinase est mesurée sur un substrat ne comportant qu'une seule sérine phosphorylable. Il s'agit d'une construction GST en fusion avec la queue de l'histone H3 (avec la sérine 10). Un substrat contrôle est également utilisé où la sérine 10 est remplacée par une alanine. Les puits 1, 4 et 7 contiennent aurora-A recombinante purifiée est utilisée. Les puits 2,5 et 8 contienent aurora-A recombinante immunoprécipitée et fixée à l'anticorps et à la protéine A Sepharose. Les puits3 et 6 ne contiennent pas de kinase. Les puits 3, 4 et 5 contiennent le substrat phosphorylable GST-H3(S) et les puits 6, 7 et 8 le substrat non phosphorylable GST-H3(S/A) pour les kinases.

## Revendications

1. Anticorps monoclonal anti-aurora-A reconnaissant spécifiquement la kinase aurora-A humaine et murine, et ayant les propriétés suivantes :
* il peut se fixer sur les membranes contenant la protéine aurora-A humaine ou murine,
* il permet de détecter, et, le cas échéant, de purifier, la protéine aurora-A humaine et murine par immunoprécipitation
* il permet la coloration des tissus biologiques où est sécrétée la protéine aurora-A, et
* il n'inhibe pas l'activité enzymatique de la protéine aurora-A humaine et murine, ledit anticorps monoclonal étant tel qu'obtenu par :
- cinq injections espacées de quinze jours à des souris de protéine kinase aurora-A recombinante produite par des bactéries *E.Coli* transformées avec un vecteur d'expression bactérien dans le génome duquel a été inséré l'ADN_{c} humain codant pour aurora-A, sacrifice des dites souris, et fusion entre des cellules de la rate de ces souris et des cellules de hamster immortalisées en culture pour obtenir des hybridomes,
- criblage des hybridomes produisant un anticorps capable d'immunoprécipiter la protéine recombinante utilisée pour l'immunisation des souris lors de l'étape précédente et récupération des hybridomes positifs après ce premier crible,
- criblage des hybridomes récupérés à l'étape précédente, produisant un anticorps capable d'immunoprécipiter la protéine endogène aurora-A à partir d'un extrait de cellules humaines HeLa en culture et récupération des hybridomes positifs après ce deuxième crible,
- criblage des hybridomes récupérés à l'étape précédente, produisant un anticorps capable de reconnaître en immunofluorescence indirecte les centrosomes et les pôles du fuseau mitotique de cellules humaines en culture, et récupération des hybridomes positifs après ce troisième crible,
- criblage des hybridomes récupérés à l'étape précédente, produisant un anticorps capable d'immunoprécipiter la protéine endogène aurora-A de souris à partir d'un extrait de cellules murines en culture et récupération des hybridomes positifs après ce quatrième crible,
- criblage des hybridomes récupérés à l'étape précédente, produisant un anticorps capable de reconnaître en immunofluorescence indirecte les centrosomes et les pôles du fuseau mitotique de cellules murines en culture,
- récupération et purification par clonage d'un hybridome positif après l'étape de criblage précédente, et produisant un anticorps monoclonal possédant l'ensemble des propriétés définies ci-dessus.

2. Anticorps monoclonal selon la revendication 1, encore désigné anticorps 35C1, tel que secrété par l'hybridome déposé à la Collection Nationale de Cultures de Microorganismes (CNCM) de l'Institut Pasteur sous le numérol-3050.

3. Utilisation d'un anticorps monoclonal tel que défini dans l'une quelconque des revendications 1 et 2, pour la mise en oeuvre d'une méthode de diagnostic in vitro, ou de pronostic de cancers chez l'homme ou l'animal.

4. Utilisation d'un anticorps monoclonal selon la revendication 3, pour la mise en oeuvre d'une méthode de diagnostic in vitro, ou de pronostic, de tumeurs solides, tels que les cancers du sein, les cancers gastriques et les cancers colorectaux.

5. Utilisation selon l'une quelconque des revendications 3 et 4, en association avec un marqueur de prolifération cellulaire, tel qu'un marqueur de la protéine PCNA.

6. Méthode de diagnostic in vitro, ou de pronostic, de cancers tels que définis dans la revendication 4, chez l'homme ou l'animal, **caractérisée en ce qu'**elle comprend :
- la mise en présence d'un anticorps monoclonal selon la revendication 1 ou 2, avec un échantillon biologique prélevé chez un individu, ledit anticorps étant le cas échéant fixé sur un support solide,
- la détection, et le cas échéant la quantification, de la protéine aurora-A susceptible d'être présente dans l'échantillon biologique à l'aide de réactifs marqués, notamment d'anticorps marqués, reconnaissant soit l'anticorps monoclonal lié à ladite protéine aurora-A, soit la protéine aurora-A liée audit anticorps monoclonal dans les complexes formés lors de l'étape précédente entre l'anticorps monoclonal et la protéine aurora-A susceptible d'être présente dans l'échantillon biologique, et ce, le cas échéant, après rinçage approprié du support solide.

7. Méthode selon la revendication 6, **caractérisée en ce que** la détermination d'une quantité de protéine aurora-A inférieure ou supérieure aux valeurs physiologiques normales dans l'échantillon biologique, témoigne respectivement d'un bon ou d'un mauvais pronostic du cancer diagnostiqué.

8. Kit pour la mise en oeuvre d'une méthode de diagnostic selon la revendication 6 ou 7, **caractérisé en ce qu'**il comprend :
- un anticorps monoclonal anti-aurora A selon la revendication 1 ou 2,
- le cas échéant, un marqueur de prolifération cellulaire, tel qu'un marqueur de la protéine PCNA, notamment un anticorps anti-PCNA.

9. Utilisation d'un anticorps défini dans l'une quelconque des revendication 1 et 2, pour la préparation de médicaments destinés au traitement de cancers, tels que les cancers du sein, les cancers colorectaux et les cancers gastriques.

10. Composition pharmaceutique contenant un anticorps selon dans l'une quelconque des revendications 1 et 2, en association avec un vecteur pharmaceutiquement acceptable.

11. Procédé de préparation d'un anticorps monoclonal anti-aurora A selon l'une quelconque des revendications 1 et 2, **caractérisé en ce qu'**il comprend les étapes suivantes :
- cinq injections espacées de quinze jours à des souris de protéine kinase aurora-A recombinante produite par des bactéries *E.Coli* transformées avec un vecteur d'expression bactérien dans le génome duquel a été inséré l'ADN_{c} humain codant pour aurora-A, sacrifice des dites souris, et fusion entre des cellules de la rate de ces souris et des cellules de hamster immortalisées en culture pour obtenir des hybridomes,
- criblage des hybridomes produisant un anticorps capable d'immunoprécipiter la protéine recombinante utilisée pour l'immunisation des souris lors de l'étape précédente et récupération des hybridomes positifs après ce premier crible,
- criblage des hybridomes récupérés à l'étape précédente, produisant un anticorps capable d'immunoprécipiter la protéine endogène aurora-A à partir d'un extrait de cellules humaines HeLa en culture et récupération des hybridomes positifs après ce deuxième crible,
- criblage des hybridomes récupérés à l'étape précédente, produisant un anticorps capable de reconnaître en immunofluorescence indirecte les centrosomes et les pôles du fuseau mitotique de cellules humaines en culture, et récupération des hybridomes positifs après ce troisième crible,
- criblage des hybridomes récupérés à l'étape précédente, produisant un anticorps capable d'immunoprécipiter la protéine endogène aurora-A de souris à partir d'un extrait de cellules murines en culture et récupération des hybridomes positifs après ce quatrième crible,
- criblage des hybridomes récupérés à l'étape précédente, produisant un anticorps capable de reconnaître en immunofluorescence indirecte les centrosomes et les pôles du fuseau mitotique de cellules murines en culture,
- récupération et purification par clonage d'un hybridome positif après l'étape de criblage précédente, et produisant un anticorps monoclonal possédant l'ensemble des propriétés définies dans la revendication 1.

## Claims

1. An anti-aurora-A monoclonal antibody specifically recognizing human and murine aurora-A kinase, and having the following properties:
* it can be fixed on the membranes containing the human or murine aurora-A protein,
* it allows detection, and, if appropriate, purification, of the human and murine aurora-A protein by immunoprecipitation,
* it allows the staining of biological tissues where the aurora-A protein is secreted, and
* it does not inhibit the enzymatic activity of the human and murine aurora-A protein,
said monoclonal antibody being as obtained by:
- five injections spread over fifteen days to mice of recombinant aurora-A protein kinase produced by *E. coli* bacteria transformed with a bacterial expression vector in the genome of which the human cDNA coding for aurora-A has been inserted, sacrifice of said mice, and fusion between cells of the spleen of these mice and hamster cells immortalized in culture in order to obtain hybridomas,
- screening of the hybridomas producing an antibody capable of immunoprecipitating the recombinant protein used for the immunization of the mice during the preceding stage, and recovery of the positive hybridomas after this first screening,
- screening of the hybridomas recovered in the preceding stage, producing an antibody capable of immunoprecipitating the endogenous aurora-A protein from an extract of human HeLa cells in culture, and recovery of the positive hybridomas after this second screening,
- screening of the hybridomas recovered in the preceding stage, producing an antibody capable of recognizing by indirect immunofluorescence the centrosomes and the poles of the mitotic spindle of human cells in culture, and recovery of the positive hybridomas after this third screening,
- screening of the hybridomas recovered in the preceding stage, producing an antibody capable of immunoprecipitating the endogenous aurora-A protein of mice from an extract of murine cells in culture, and recovery of the positive hybridomas after this fourth screening,
- screening of the hybridomas recovered in the preceding stage, producing an antibody capable of recognizing by indirect immunofluorescence the centrosomes and the poles of the mitotic spindle of murine cells in culture,
- recovery and purification by cloning of a positive hybridoma after the preceding screening stage, and production of a monoclonal antibody possessing all of the properties defined above.

2. Monoclonal antibody according to claim 1, also called 35C1 antibody, as secreted by the hybridoma deposited at the Collection Nationale de Cultures de Microorganismes (CNCM) of the Institut Pasteur under the number I-3050.

3. Use of a monoclonal antibody as defined in claim 1 or 2, for the implementation of a method for *in vitro* diagnostic, or prognostic of cancers in humans or animals.

4. Use of a monoclonal antibody according to claim 3, for *in vitro* diagnostic or prognostic method for solid tumours such as breast cancers, stomach cancers and colorectal cancers.

5. Use of a monoclonal antibody according to claim 3 or 4, also comprising the use of a cell proliferation marker, such as a marker of the PCNA protein.

6. An *in vitro* diagnostic or prognostic method for cancers as defined in claim 4, in humans or animals, **characterized in that** it comprises:
- placing a monoclonal antibody according to claim 1 or 2, in the presence of a biological sample taken from an individual, said antibody if appropriate being fixed on a solid support,
- the detection, and if appropriate the quantitation, of the aurora-A protein which may be present in the biological sample using marked reagents, in particular marked antibodies, recognizing either the monoclonal antibody linked to said aurora-A protein, or the aurora-A protein linked to said monoclonal antibody in the complexes formed during the preceding stage between the monoclonal antibody and the aurora-A protein which may be present in the biological sample, this, if necessary, after appropriate rinsing of the solid support.

7. Method according to claim 6, **characterized in that** the determination of a quantity of aurora-A protein lower than or greater than the normal physiological values in the biological sample, shows respectively a good or a poor prognosis for the diagnosed cancer.

8. Kit for the implementation of a diagnostic method according to claim 6 or 7, **characterized in that** it comprises:
- an anti-aurora-A monoclonal antibody according to claim 1,
- if appropriate, a cell proliferation marker, such as a marker of the PCNA protein, in particular an anti-PCNA antibody.

9. Use of an antibody as defined in claim 1 or 2, for the preparation of a drug intended for the treatment of cancers, such as breast cancers, colorectal cancers and stomach cancers.

10. Pharmaceutical composition containing an antibody according to claim 1 or 2, in combination with a pharmaceutically acceptable vector.

11. Method for the preparation of an anti-aurora-A monoclonal antibody according to claim 1 or 2, **characterized in that** it comprises the following stages:
- five injections spread over fifteen days to mice of recombinant aurora-A protein kinase produced by *E. coli* bacteria transformed with a bacterial expression vector in the genome of which the human cDNA coding for aurora-A has been inserted, sacrifice of said mice, and fusion between cells of the spleen of these mice and hamster cells immortalized in culture in order to obtain hybridomas,
- screening of the hybridomas producing an antibody capable of immunoprecipitating the recombinant protein used for the immunization of the mice during the preceding stage, and recovery of the positive hybridomas after this first screening,
- screening of the hybridomas recovered in the preceding stage, producing an antibody capable of immunoprecipitating the endogenous aurora-A protein from an extract of human HeLa cells in culture, and recovery of the positive hybridomas after this second screening,
- screening of the hybridomas recovered in the preceding stage, producing an antibody capable of recognizing by indirect immunofluorescence the centrosomes and the poles of the mitotic spindle of human cells in culture, and recovery of the positive hybridomas after this third screening,
- screening of the hybridomas recovered in the preceding stage, producing an antibody capable of immunoprecipitating the endogenous aurora-A protein of mice from an extract of murine cells in culture, and recovery of the positive hybridomas after this fourth screening,
- screening of the hybridomas recovered in the preceding stage, producing an antibody capable of recognizing by indirect immunofluorescence the centrosomes and the poles of the mitotic spindle of murine cells in culture,
- recovery and purification by cloning of a positive hybridoma after the preceding screening stage, and production of a monoclonal antibody possessing all of the properties defined in claim 1.

## Patentansprüche

1. Monoklonaler Anti-Aurora-A-Antikörper, der die humane und murine Kinase Aurora-A spezifisch erkennt und die folgenden Eigenschaften aufweist:
- er kann sich an Membranen, die das humane oder murine Protein Aurora-A aufweisen, binden,
- er ermöglicht den Nachweis und gegebenenfalls die Reinigung des humanen oder murinen Proteins Aurora-A durch Immunfällung,
- er ermöglicht die Färbung biologischer Gewebe, wo das Protein Aurora-A sezerniert wird, und
- er hemmt die Enzymaktivität des humanen oder murinen Proteins Aurora-A nicht,
wobei der monoklonale Antikörper erhalten ist durch:
- fünf über fünfzehn Tage verteilte Injektionen von rekombinanter Proteinkinase Aurora-A in Mäuse, die von *E.coli*-Bakterien produziert wurde, die mit einem bakteriellen Expressionsvektor modifiziert wurden, in dessen Genom menschliche cDNA, die für Aurora-A codiert, inseriert wurde, das Töten der Mäuse und das Fusionieren von Milzzellen der Mäuse und in Kultur immortalisierten Hamsterzellen, um Hybridome zu erhalten,
- das Screenen der Hybridome, die einen Antikörper produzieren, der zur Immunfällung des rekombinanten Proteins, das zur Immunisierung der Mäuse im vorangegangenen Schritt verwendet wurde, imstande ist, und das Gewinnen der positiven Hybridome nach diesem ersten Screen,
- das Screenen der im vorangegangenen Schritt gewonnenen Hybridome, die einen Antikörper produzieren, der zur Immunfällung des endogenen Proteins Aurora-A ausgehend von einem humanen HeLa-Zellextrakt in Kultur imstande ist, und das Gewinnen der positiven Hybridome nach diesem zweiten Screen,
- das Screenen der im vorangegangenen Schritt gewonnenen Hybridome, die einen Antikörper produzieren, der zur Erkennung in indirekter Immunfluoreszenz der Centrosome und der Pole des Spindelapparats von humanen Zellen in Kultur imstande ist, und das Gewinnen der positiven Hybridome nach diesem dritten Screen,
- das Screenen der im vorangegangenen Schritt gewonnenen Hybridome, die einen Antikörper produzieren, der zur Immunfällung des endogenen Maus-Proteins Aurora-A ausgehend von einem murinen Zellextrakt in Kultur imstande ist, und das Gewinnen der positiven Hybridome nach diesem vierten Screen,
- das Screenen der im vorangegangenen Schritt gewonnenen Hybridome, die einen Antikörper produzieren, der zur Erkennung in indirekter Immunfluoreszenz der Centrosome und der Pole des Spindelapparats von murinen Zellen in Kultur imstande ist,
- das Gewinnen und das Reinigen durch Klonieren eines positiven Hybridoms nach dem vorangegangenen Screeningschritt, das einen monoklonalen Antikörper mit allen oben definierten Eigenschaften produziert.

2. Monoklonaler Antikörper nach Anspruch 1, auch als Antikörper 35C1 bezeichnet, der vom Hybridom sezerniert wird, das in der Collection Nationale de Cultures de Microorganismes (CNCM) des Institut Pasteur mit der Nummer 1-3050 hinterlegt ist.

3. Verwendung eines monoklonalen Antikörpers nach einem der Ansprüche 1 oder 2 zur Durchführung eines Verfahrens der In-vitro-Diagnose oder der Prognose von Krebs bei Menschen oder Tieren.

4. Verwendung eines monoklonalen Antikörpers nach Anspruch 3 zur Durchführung eines Verfahrens der In-vitro-Diagnose oder der Prognose von soliden Tumoren, wie etwa Brustkrebs, Magenkrebs oder Kolorektalkrebs.

5. Verwendung nach einem der Ansprüche 3 oder 4 gemeinsam mit einem Zellproliferationsmarker, wie etwa einem Marker des Proteins PCNA.

6. Verfahren zur In-vitro-Diagnose oder Prognose von Krebs, der in Anspruch 4 definiert ist, bei Menschen und Tieren, **dadurch gekennzeichnet, dass** es Folgendes umfasst:
- das In-Kontakt-Bringen eines monoklonalen Antikörpers nach Anspruch 1 oder 2 mit einer biologischen Probe, die von einem Individuum entnommen wurde, wobei der Antikörper gegebenenfalls an einem festen Träger angebracht ist,
- das Nachweisen und gegebenenfalls das Quantifizieren des Proteins Aurora-A, das eventuell in der biologischen Probe gegenwärtig ist, mithilfe von markierten Reagenzien, insbesondere von markierten Antikörpern, die entweder den monoklonalen Antikörper, der an das Protein Aurora-A gebunden ist, oder das Protein Aurora-A, das an den monoklonalen Antikörper gebunden ist, in den Komplexen, die im vorangegangenen Schritt zwischen dem monoklonalen Antikörper und dem Protein Aurora-A, das eventuell in der biologischen Probe gegenwärtig ist, gebildet wurden, erkennen, und dies gegebenenfalls nach einer geeigneten Spülung des festen Trägers.

7. Verfahren nach Anspruch 6, **dadurch gekennzeichnet, dass** die Bestimmung einer Menge an Protein Aurora-A unter oder über den physiologischen Normalwerten in der biologischen Probe auf eine gute bzw. eine schlechte Prognose für den diagnostizierten Krebs hinweist.

8. Kit zur Durchführung eines Diagnoseverfahrens nach Anspruch 6 oder 7, **dadurch gekennzeichnet, dass** es Folgendes umfasst:
- einen monoklonalen Anti-Aurora-A-Antikörper nach Anspruch 1 oder 2,
- gegebenenfalls einen Zellproliferationsmarker, wie etwa einen Marker des Proteins PCNA, insbesondere einen Anti-PCNA-Antikörper.

9. Verwendung eines Antikörpers nach einem der Ansprüche 1 oder 2 zur Herstellung von Medikamenten zur Behandlung Krebs, wie etwa Brustkrebs, Kolorektalkrebs oder Magenkrebs.

10. Pharmazeutische Zusammensetzung, die einen Antikörper nach einem der Ansprüche 1 oder 2 enthält, gemeinsam mit einem pharmazeutisch annehmbaren Vektor.

11. Verfahren zur Herstellung eines monoklonalen Anti-Aurora-A-Antikörpers nach einem der Ansprüche 1 oder 2, **dadurch gekennzeichnet, dass** es die folgenden Schritte umfasst:
- fünf über fünfzehn Tage verteilte Injektionen von rekombinanter Proteinkinase Aurora-A in Mäuse, die von *E.coli*-Bakterien produziert wurde, die mit einem bakteriellen Expressionsvektor modifiziert wurden, in dessen Genom menschliche cDNA, die für Aurora-A codiert, inseriert wurde, das Töten der Mäuse und das Fusionieren von Milzzellen der Mäuse und in Kultur immortalisierten Hamsterzellen, um Hybridome zu erhalten,
- das Screenen der Hybridome, die einen Antikörper produzieren, der zur Immunfällung des rekombinanten Proteins, das zur Immunisierung der Mäuse im vorangegangenen Schritt verwendet wurde, imstande ist, und das Gewinnen der positiven Hybridome nach diesem ersten Screen,
- das Screenen der im vorangegangenen Schritt gewonnenen Hybridome, die einen Antikörper produzieren, der zur Immunfällung des endogenen Proteins Aurora-A ausgehend von einem humanen HeLa-Zellextrakt in Kultur imstande ist, und das Gewinnen der positiven Hybridome nach diesem zweiten Screen,
- das Screenen der im vorangegangenen Schritt gewonnenen Hybridome, die einen Antikörper produzieren, der zur Erkennung in indirekter Immunfluoreszenz der Centrosome und der Pole des Spindelapparats von humanen Zellen in Kultur imstande ist, und das Gewinnen der positiven Hybridome nach diesem dritten Screen,
- das Screenen der im vorangegangenen Schritt gewonnenen Hybridome, die einen Antikörper produzieren, der zur Immunfällung des endogenen Maus-Proteins Aurora-A ausgehend von einem murinen Zellextrakt in Kultur imstande ist, und das Gewinnen der positiven Hybridome nach diesem vierten Screen,
- das Screenen der im vorangegangenen Schritt gewonnenen Hybridome, die einen Antikörper produzieren, der zur Erkennung in indirekter Immunfluoreszenz der Centrosome und der Pole des Spindelapparats von murinen Zellen in Kultur imstande ist,
- das Gewinnen und das Reinigen durch Klonieren eines positiven Hybridoms nach dem vorangegangenen Screeningschritt, das einen monoklonalen Antikörper mit allen oben definierten Eigenschaften produziert.
